# EUROPÄISCHE PATENTANMELDUNG

(11) **EP 1 607 026 A2**
(43) Veröffentlichungstag der Anmeldung: **21.12.2005**
(21) Anmeldenummer: 05006526.7
(22) Anmeldetag: 24.03.2005
(51) Int. Cl.: A47C 27/00, A01M 1/20, A61L 2/00

(54) **Vorrichtung zur Verbringung von Anti-Milben-Mittel in eine Matratze**

(30) Priorität: 31.03.2004 DE 102004016537
(71) Anmelder: MATRIX GmbH, 76185 Karlsruhe (DE)
(72) Erfinder:
(74) Vertreter: Petersen, Frank

(57) **Zusammenfassung**

Die Erfindung betrifft eine Vorrichtung zur Verbringung von Anti-Milben-Mittel in eine Matratze.

Um sicherstellen zu können, dass eine Matratze (1) nicht nur oberflächlich mit einem Anti-Milben-Mittel besprüht wird, wird vorgeschlagen, eine entsprechende Vorrichtung mit Bauelementen (4) zu versehen, die wenigstens eine Hohlnadel (9) aufweisen, mit denen ein Außenbezug einer Matratze (1) durchstochen wird, so dass mit diesen Vorrichtungen eine Matratze (1) mit Anti-Milben-Mittel zu impfen ist.

## Beschreibung

Die Erfindung betrifft eine Vorrichtung zur Verbringung von Anti-Milben-Mittel in eine Matratze.

In zunehmendem Maße leiden Allergiker heute unter Hausstaub. Es ist dabei bekannt, dass derartiger Hausstaub insbesondere durch die Ausscheidungen von Hausstaubmilben allergen wird, die zum Beispiel in Matratzen zu finden sind, wo sie sich von Hautschuppen etc. ernähren. Um diese Hausstaubmilben zu beseitigen, sind bereits verschiedenste Möglichkeiten vorgeschlagen worden. Insbesondere gibt es dabei Vorschläge, die Matratzen zu reinigen, wobei die Matratzen in entsprechende Reinigungsmaschinen eingebracht werden.

Es hat sich aber herausgestellt, dass gerade die Hausstaubmilben auch höheren Temperaturen und Drücken über längere Zeit widerstehen können, so dass letztlich dazu übergegangen wird, Matratzen mit einem Anti-Milben-Mittel zu behandeln. Hierzu wird bisher üblicherweise vorgeschlagen, eine Düse über die Düse zu halten und das üblicherweise flüssige Anti-Milben-Mittel auf die Matratze aufzusprühen.

Hierdurch wird aber nur eine sehr oberflächliche Wirkung erreicht.

Um tiefer liegende Schichten der Matratze zu erreichen, wird vorgeschlagen, die Düse auf die Matratze aufzusetzen und durch diese hindurch ein entsprechendes Anti-Milben-Mittel in die Matratze einzudüsen.

Dabei hat es sich jetzt aber als problematisch herausgestellt, dass bei einem derartigen Vorgehen das Anti-Milben-Mittel nicht sehr tief in die Matratze eindringt, so dass der Behandlungserfolg mit dem Anti-Milben-Mittel als fraglich anzusehen ist.

Unter diesem Aspekt stellt sich die Aufgabe, die bisher bekannten Vorrichtungen zu verbessern, um sicherstellen zu können, dass Anti-Milben-Mittel tatsächlich auch in tiefere Bereiche einer Matratze eingebracht werden kann.

Diese Aufgabe wird erfindungsgemäß dadurch gelöst, dass die Vorrichtung zur Verbringung von Anti-Milben-Mittel in eine Matratze ein Bauelement aufweist, das mit wenigstens einer Hohlnadel versehen ist, mit der der Außenbezug der Matratze durchstochen wird.

Die Erfindung hat den Vorteil, dass die als besonders dicht anzusehende äußere Bespannung einer Matratze durch die Hohlnadel überwunden wird und das Anti-Milben-Mittel dann durch die Hohlnadel hindurch in die unter dem Außenbezug liegenden Schichten eingebracht werden kann. Hier kann es sich dann entsprechend verteilen.

Hierbei ist auch zu berücksichtigen, dass das vordere Ende der Hohlnadel bei einem entsprechend hohen Druck des durch die Hohlnadel hindurch gedüsten Anti-Milben-Mittels auch als Düse wirkt, so dass unterhalb des Außenbezuges der Matratze das Anti-Milben-Mittel nicht als Strahl sondern als Nebel o. ä. in die Matratze bzw. den Matratzenkern eingebracht wird. Hierdurch ist eine besonders gleichmäßige Verteilung des üblicherweise flüssigen Anti-Milben-Mittels zu erreichen.

Um den entsprechend hohen Druck herstellen zu können, wird vorgeschlagen, dass die Hohlnadel mit einer Kammer kommuniziert, aus der mittels eines Kolbens Anti-Milben-Mittel durch die Hohlnadel gedrückt wird. Man hat somit den Vorteil, einen entsprechend hohen Druck aufbauen zu können.

Vorzugsweise ist die Kammer strömungstechnisch über ein Ventil von der Hohlnadel getrennt. Dieses kann insbesondere als ein doppeltes Rückschlagventil so ausgeführt werden, dass die Kammer zur Wiederbefüllung mit einem Reservoir verbindbar ist, das als Tank das Anti-Milben-Mittel enthält, bei gleichzeitiger Abschaltung der Verbindung zur Hohlnadel. Wenn dann der Kolben innerhalb der Kammer wieder Druck aufbaut, wird die Klappe des Rückschlagventils, die zu dem Reservoir führt, geschlossen und gleichzeitig das Rückschlagventil zur Hohlnadel geöffnet, so dass ein weiterer Schub Anti-Milben-Mittel durch die Hohlnadel in die Matratze einbringbar ist. Dies ist eine besonders betriebssichere Bauweise.

Weiterhin ist noch zu berücksichtigen, dass die Hohlnadel über einen Halter auswechselbar an dem Bauelement befestigt ist, um unterschiedliche Polstermaterialien berücksichtigen zu können. Hierbei ist bei einer Matratze zu beachten, ob es eine Federkern- oder eine Latexmatratze ist, wie die Kerne mit weiteren Polstermaterialien (Wolle, Rosshaar, Polsterwatte etc.) belegt sind usw.

Die Verbindung von Halter und Hohlnadel kann beispielsweise über einen Bajonettverschluss erfolgen, vorzugsweise ist hier aber ein Gewinde vorgesehen, da dieses gegebenenfalls eine Selbsthemmung hat und somit ein unbeabsichtigtes Lösen der Hohlnadel von dem Bauelement zu verhindern ist.

Die Vorrichtung kann insbesondere als Handgerät ausgebildet sein. Dann hat sie insbesondere eine auf den Kolben wirkende Abzugsvorrichtung und einen Griff.

Es ist aber auch möglich, die Vorrichtung als Einbauelement einer größeren Maschine mit mehreren Bauelementen auszuführen die an einer Halterung angeordneten sind und über eine gemeinsame maschinelle Betätigung verfügen. Hiermit kann eine zeitsparende Bearbeitung einer Matratze beispielsweise über ihre gesamte Breite in einem Arbeitsgang erfolgen.

Vorzugsweise ist dabei die Halterung so ausgebildet, dass zwei einander im Wesentlichen gegenüberliegende Träger vorgesehen sind, an denen die Bauelemente in einander zugewandter Orientierung der Hohlnadeln angeordnet sind.

Damit kann eine Matratze gleichzeitig von ihrer Ober- und Unterseite mit einem entsprechenden Anti-Milben-Mittel bearbeitet werden.

Weitere Vorteile und Merkmale der Erfindung ergeben sich aus der nachfolgenden Beschreibung eines Ausführungsbeispiels. Dabei zeigt:
- Figur 1: die Prinzipskizze einer Vorrichtung zur Verbringung von Anti-Milben-Mittel in eine Matratze;
- Figur 2: eine Prinzipskizze für ein Bauelement mit einer Hohlnadel als Teil einer Vorrichtung gemäß Figur 1.

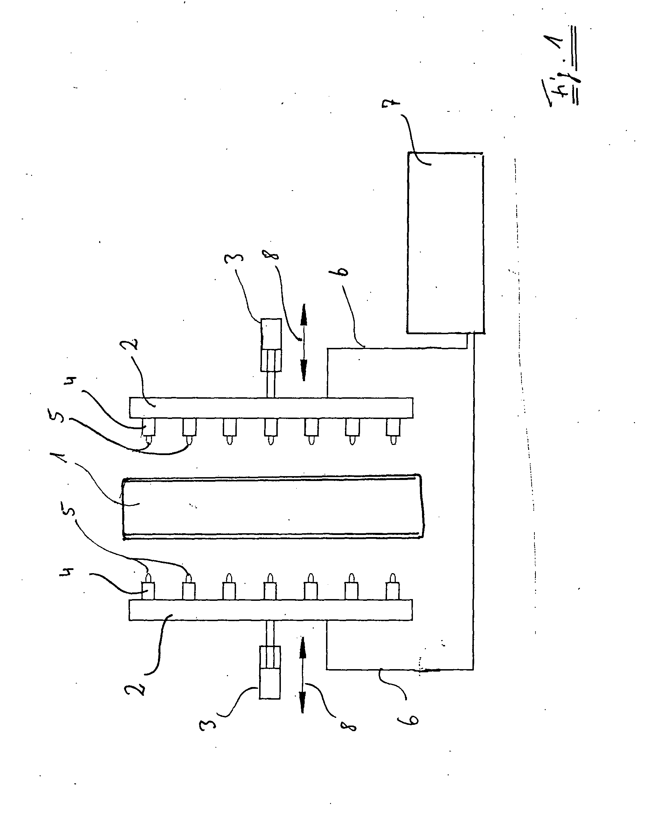

In der Figur 1 erkennt man die Prinzipskizze einer Vorrichtung zur Verbringung von Anti-Milben-Mitteln in eine Matratze. Diese Vorrichtung ist Teil einer größeren Matratzenreinigungsanlage, die hier im ganzen aber nicht dargestellt ist.

Bei dieser Vorrichtung werden an eine Matratze 1 zwei Träger 2 über pneumatische oder hydraulische Zylinder-Kolben-Aggregate 3 herangeführt. Auf diesen Trägern sitzen einander im Wesentlichen gegenüberliegend Bauelemente 4, die so von entgegengesetzten Seiten an die Matratze 1 herangefahren werden. Die Bauelemente 4 sind dabei einander zugewandt und tragen an ihren einander zugewandten Spitzen 5 entweder Düsen oder aber im hier dargestellten Beispiel Hohlnadeln. Dies wird weiter unten noch näher erläutert.

Über diese Spitzen 5 wird somit, nachdem sie an die Matratze herangefahren sind, in diese ein Anti-Milben-Mittel eingesprüht, das beispielsweise auf einer Nehmölbasis ist. Dieses Anti-Milben-Mittel ist üblicherweise flüssig und wird den Spitzen über Leitungen 6 aus einem zentralen Tank 7 zugeführt.

Nachdem das entsprechende Anti-Milben-Mittel in die Matratze eingebracht ist, werden die Träger 2 über die Zylinder-Kolben-Aggregate 3 zurückgezogen und nachdem eine Verschiebebewegung zwischen Matratze und Trägern erfolgt ist, wieder an die Matratze herangeführt, um den nächsten Streifen der Matratze mit Anti-Milben-Mittel zu beaufschlagen. Die Träger 2 führen somit permanent eine Bewegung aufeinander zu und voneinander weg aus, wie sie durch die Doppelpfeile 8 angedeutet werden.

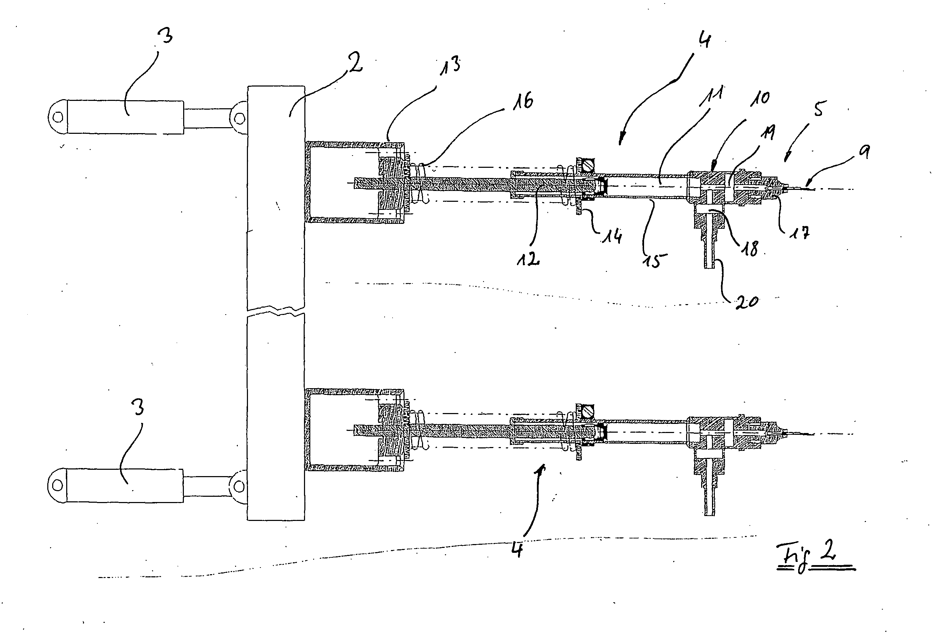

In der Figur 2 ist ein entsprechender Träger 2 mit darauf aufsitzenden Bauelementen 4 detaillierter dargestellt.

Man erkennt, dass die Bauelemente 4 an ihren dem Träger 2 abgewandten Enden bzw. Spitzen 5 Hohlnadeln 9 tragen. Diese Hohlnadeln kommunizieren über einen Anschluss, insbesondere ein Ventil 10, mit einer Kammer 11. In diese Kammer 11 ist ein Kolben 12 einfahrbar, wobei dann in der Kammer 11 befindliche Flüssigkeit durch das Ventil 10 und die Hohlnadel 9 hindurchtritt.

Der Kolben 12 ist über eine Befestigung 13 mit dem Träger 2 verbunden. Zwischen der Befestigung 13 und einem Flansch 14, der an der Wand 15 der Kammer 11 angebracht ist, findet sich hier noch eine Druckfeder 16.

Das gesamte Bauelement 4 fungiert dabei wie folgt:

Bei Vorschieben des Trägers 2 durch die Zylinder-Kolben-Aggregate 3 werden die Hohlnadeln 9 durch die Bespannung einer (nicht dargestellten) Matratze hindurchgedrückt, bis die Halter 17 der Hohlnadel 9 in Anlage an die Matratze kommen.

Bei einem weiteren Vorfahren des Trägers 2 über die Zylinder-Kolben-Aggregate 3 wird dann der Kolben 12 in die Kammer 11 hineingedrückt, wobei wie oben erwähnt die in der Kammer 11 befindliche Flüssigkeit durch die Hohlnadel 9 in die Matratze eingebracht wird. Dabei schließt im Ventil 10 ein Rückschlagventil 18, während ein zweites Rückschlagventil 19 zur Hohlnadel hin öffnet.

Gleichzeitig wird bei dem Vordrücken des Kolbens 12 in die Kammer 11 die Druckfeder 16 gespannt.

Beim Zurückziehen des Trägers 2 über die Zylinder-Kolben-Aggregate 3 wird sich dann die Feder 16 wieder entspannen, wobei sie dann bezüglich des Kolbens 12 über den Flansch 14 die Wand 15 der Kammer 11 in die entgegengesetzte Richtung bewegen wird, womit die Kammer 11 wieder mit Anti-Milben-Mittel gefüllt wird. Hierzu wird das zweite Rückschlagventil 19 zur Hohlnadel geschlossen und das Rückschlagventil 18 öffnet sich, so dass über den Ansatzstutzen 20, der mit der Leitung 6 und dem Tank 7 verbunden ist, wieder Anti-Milben-Mittel in die Kammer 11 nachströmen kann.

Durch ein erneutes Aufsetzen der Hohlnadeln 9 auf benachbarten Punkte der Matratze 1 wird dann dieser Vorgang entsprechend wiederholt.

Man hat somit eine Möglichkeit, eine Matratze in einem quasi-kontinuierlichem Durchgang verständig mit Anti-Milben-Mittel zu versehen, um somit eine sichere Bekämpfung der Hausstaubmilben in den Matratzen bewirken zu können.

Es sei an dieser Stelle noch erwähnt, dass der Halter 17 für die Hohlnadel 9 über ein Gewinde oder einen Bajonettverschluss vom Ventilkörper 10 gelöst werden kann, um frische oder andere Hohlnadeln 9 aufzusetzen.

Des weiteren sei erwähnt, dass der Träger 2 beispielsweise auch an einem manuell zu betätigenden Griff montiert sein kann, über den dann bei im Prinzip gleicher Funktionsweise wie oben beschrieben eine Matratze manuell mit entsprechendem Anti-Milben-Mittel zu impfen ist. Auch ein manuelles Einspritzen oder Einblasen von Anti-Milben-Mittel, gegebenenfalls unter Verzicht auf den Träger 2 liegt, also im Rahmen der Erfindung.

## Patentansprüche

1. Vorrichtung zur Verbringung von Anti-Milben-Mittel in eine Matratze (1),
**dadurch gekennzeichnet,**
**dass** sie ein Bauelement (4) aufweist, das mit wenigstens einer Hohlnadel (9) versehen ist, zur Durchstechung eines Außenbezuges der Matratze (1).

2. Vorrichtung gemäß Anspruch 1,
**dadurch gekennzeichnet,**
**dass** die Hohlnadel (9) mit einer Kammer (11) kommuniziert, aus der mittels eines Kolbens (12) Anti-Milben-Mittel durch die Hohlnadel (9) gedrückt wird.

3. Vorrichtung gemäß Anspruch 2,
**dadurch gekennzeichnet,**
**dass** die Kammer (11) strömungstechnisch über ein Ventil (10; 18, 19) von der Hohlnadel (9) getrennt und zur Wiederbefüllung mit einem Tank (7) verbindbar ist.

4. Vorrichtung gemäß Anspruch 1,
**dadurch gekennzeichnet,**
**dass** die Hohlnadel (9) über einen Halter (17) auswechselbar ist.

5. Vorrichtung gemäß Anspruch 1,
**dadurch gekennzeichnet,**
**dass** die Vorrichtung einen mit einer auf den Kolben (12) wirkenden Abzugsvorrichtung (2) versehenen Griff aufweist.

6. Vorrichtung gemäß Anspruch 1,
**dadurch gekennzeichnet,**
**dass** die Vorrichtung als Einbauelement mehrere an einer Halterung (2) angeordnete, über eine maschinelle Betätigung (3) verfügende Bauelemente (4) aufweist.

7. Vorrichtung gemäß Anspruch 6,
**dadurch gekennzeichnet,**
**dass** die Halterung über wenigstens zwei einander im wesentlichen gegenüberliegende Träger (2) verfügt, an denen Bauelemente (4) in einander zugewandter Orientierung der Hohlnadeln (9) vorgesehen sind.
